# EUROPÄISCHE PATENTSCHRIFT

(11) **EP 3 752 838 B1**
(45) Veröffentlichungstag und Bekanntmachung des Hinweises auf die Patenterteilung: **03.08.2022**
(21) Anmeldenummer: 19709626.6
(22) Anmeldetag: 15.02.2019
(51) Int. Cl.: G01N 33/68

(54) **METHODE ZUR BESTIMMUNG DER TOTALEN HISTAMIN-ABBAUKAPAZITÄT IN BIOLOGISCHEN PROBEN**
METHOD FOR DETERMINING THE TOTAL HISTAMINE DEGRADATION CAPACITY IN BIOLOGICAL SAMPLES
PROCÉDÉ DE DETERMINATION DE LA CAPACITÉ TOTALE DE LA DÉGRADATION DE L'HISTAMINE DANS DES ÉCHANTILLONS BIOLOGIQUES

(30) Priorität: 16.02.2018 DE 102018103545
(43) Veröffentlichungstag der Anmeldung: 23.12.2020
(73) Patentinhaber: Frost Diagnostika GmbH, 67166 Otterstadt (DE)
(72) Erfinder: FROST, Gabriele, 67346 Speyer (DE)
(74) Vertreter: Fleuchaus, Andrea
(86) Internationale Anmeldenummer: PCT/EP2019/053869
(87) Internationale Veröffentlichungsnummer: WO 2019/158718

(56) Entgegenhaltungen:
- WO-A1-2007/056788
- MOREL A M ET AL: "IMMUNOANALYSIS OF HISTAMINE THROUGH A NOVEL CHEMICAL DERIVATIZATION", JOURNAL OF ALLERGY AND CLINICAL IMMUNO, ELSEVIER, AMSTERDAM, NL, Bd. 82, Nr. 4, 1. Oktober 1988 (1988-10-01), Seiten 646-654, XP009080391, ISSN: 0091-6749, DOI: 10.1016/0091-6749(88)90978-5
- PFISTERER M ET AL: "New diagnostic and treatment opportunities of food-intolerance caused by histamine: first clinical results", ALL, WILEY-BLACKWELL PUBLISHING LTD, UNITED KINGDOM, Bd. 62, Nr. Suppl. 83, 1. Juni 2007 (2007-06-01), Seite 333, XP009156956, ISSN: 0105-4538, DOI: 10.1111/J.1398-9995.2007.01407.X [gefunden am 2007-05-25]
- C Reiser ET AL: "Assessment and topographical analysis of the total histamine degradation capacity (THDC) at the lower GI-tract", ZEITSCHRIFT FUER GASTROENTEROLOGIE, vol. 51, no. 08, 16 August 2013 (2013-08-16), XP055579766, DE ISSN: 0044-2771, DOI: 10.1055/s-0033-1352932

## Beschreibung

Histamin, ein basisches biogenes Amin, ist eine einfache chemische Substanz mit einem Molekulargewicht von 111 Da. Die IUPAC-Bezeichnung ist 1H-Imidazol-4-Äthylamin.

Histamin ist ein wichtiger Mediator bei verschiedenen allergischen Erkrankungen, Urticaria sowie Medikamenten-Allergien bzw. -Unverträglichkeiten.

Die Konzentration von freiem Histamin wird sowohl intra- als auch extrazellulär über verschiedene Mechanismen sehr stringent geregelt, die physiologische Aktivität oder Wirksamkeit wird im menschlichen Organismus über vier verschiedene Rezeptoren gesteuert.

Eine immunologische Methode zur Quantifizierung von Histamin in flüssigen biologischen Proben oder Zellextrakten ist bspw. durch Morel et al., 1988 bekannt.

Im Organismus von Säugetieren wird Histamin primär von zwei Enzymen abgebaut: Diaminoxidase (DAO, EC 1.4.3.6) und Histamin-N-Methyltransferase (NMT, EC 2.1.1.8).

Die DAO katalysiert die oxidative Deaminierung von Histamin zu Imidazolazetaldehyd; NMT katalysiert die N-Methylierung zu N-Methylhistamin. Beide Systeme ergänzen sich, da die DAO mehr für die Regulation extrazellulär angefallenen Histamins zuständig ist und die NMT vorrangig für die intrazelluläre Regulation im Zytosol zuständig ist. Über die Regelung der beiden Enzyme ebenso wie über Mechanismen der Steuerung der Histamin-Produktion sowie der HistaminAusschüttung von Mastzellen oder des Histamin-Abbaus sind bis dato erstaunlicherweise kaum wissenschaftlich verwertbare Daten vorhanden.

Die beiden Enzyme können auch die meisten anderen natürlich vorkommenden biogenen Amine metabolisieren wie Putrescin, Cadaverin, Spermin, Spermidine, Tyramin, und Phenylethylamin, jedoch in unterschiedlichem Ausmaß.

Die absoluten Konzentrationen der verschiedenen biogenen Amine in der jeweiligen Probe bestimmen letztendlich die Kapazität der Enzyme in dieser Probe, extern zugesetztes sowie in der Probe enthaltenes Histamin abzubauen.

Bisherige klinische Erfahrungen lassen den Schluss zu, dass ein Ungleichgewicht zwischen Histamin-Freisetzung bzw. Nahrungsmittel-bedingter Histamin-Zufuhr einerseits und der Histamin-Abbaukapazität des betroffenen Systems andererseits für den Symptomkomplex der sogenannten Histamin-Intoleranz (HIT) verantwortlich ist.

Bei der Histamin-Intoleranz reagiert der Körper mit Unverträglichkeitsreaktionen auf eine erhöhte Menge an Histamin. Histamin kommt als natürlicher Stoff im Körper vor, wird aber auch vor allem durch gereifte Lebensmittel u.a. altem Käse, Gepökeltem oder alkoholischen Getränken zugeführt. Die Unverträglichkeit wird nach heutigem Verständnis durch einen Mangel oder Unterfunktion an DAO und/oder HNMT verursacht und führt somit zu einem Ungleichgewicht zwischen Zufuhr und Abbau des Histamins.

Dies ist insofern von wesentlicher Bedeutung, da die Kapazität des Abbaus von Histamin mit den Symptomen der Histamin-Intoleranz-Erkrankung zu korrelieren scheint. Überraschenderweise ist die Ausgangskonzentration oder Basalkonzentration von Histamin in biologischen Proben hohen Schwankungen unterworfen, zudem wird die Basalkonzentration - speziell bei Blutproben - durch die Prozedur der Blutabnahme und anschließender Zentrifugation stark beeinflusst und geradezu unkontrollierbar. Hinzu kommt, dassje nach Probe Histamin in unterschiedlichen Mengen aus Speicherzellen freigesetzt wird und auch so die physiologisch wirksame Basalkonzentration vor der Blutabnahme nicht eindeutig bestimmbar ist. Dieser bis dato wissenschaftlich kaum untersuchte Abbauprozess stellt eine weitere Herausforderung beim Versuch dar, eine probenspezifische Histamin-Abbaukapazität zu bestimmen. Überlagert wird dieser Effekt durch eine bis dato ebenso wenig erforschte probenspezifische unterschiedlich schnell ablaufende intrinsische Abbaukapazität von Histamin.

Die bisherige klinische Erfahrung zeigt, dass die Histamin-Abbaukapazität u.a. auch stark von den Ernährungsgewohnheiten der betroffenen Personen abhängig ist.

Mögliche Symptome bei einer Aufnahme histaminreicher Nahrung sind u.a. Hautirritationen, Kopfschmerzen, Atembeschwerden, Probleme des Verdauungstraktes und Bluthochdruck. Diese Symptome sind jedoch auch anderen Krankheitsbildern zuzuordnen. Bislang ist es daher nicht möglich eine Histamin-Intoleranz als Ursache für diese Beschwerden durch geeignete Testverfahren auszuschließen oder zu konkretisieren.

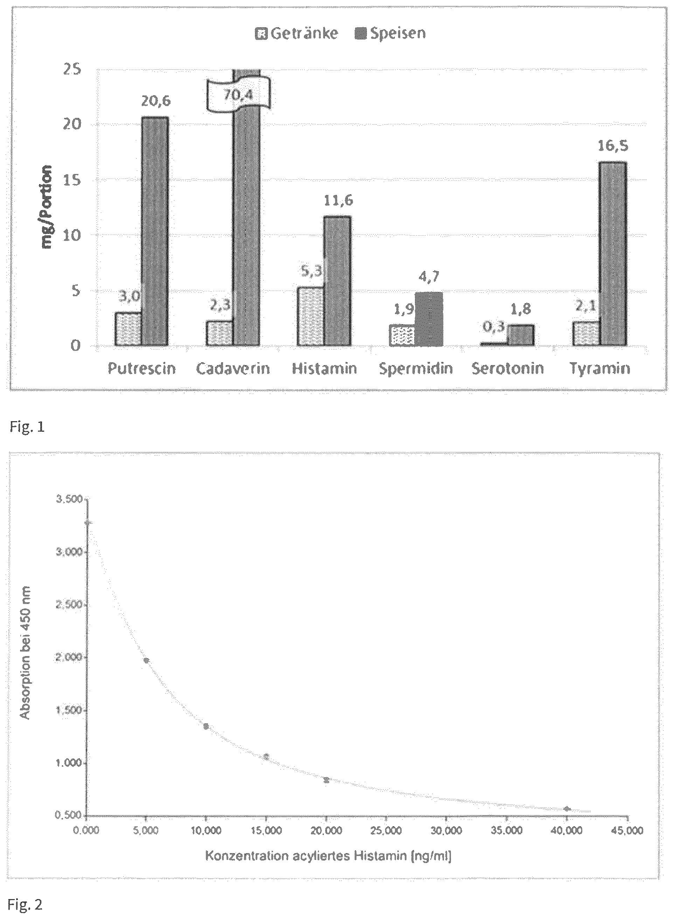

Die geschilderte Abhängigkeit der Histamin-Abbaukapazität von dem individuellen Ernährungsstatus eines Probanden mag der Tatsache geschuldet sein, dass - im Gegensatz zur weit verbreiteten Meinung - Histamin bei weitem nicht das häufigste biogene Amin in Lebensmitteln ist (siehe Fig. 1).

In der Praxis ist es de facto aber bedauerlicherweise unmöglich, einen aktuellen Einfluss des Ernährungsstatus der Probanden zu ermitteln und daher den potentiellen Einfluss anderer biogener Amine auf den Histaminabbau zu berücksichtigen.

Im Stand der Technik wurde bislang versucht, über die Aktivitätsbestimmung der DAO aus Serum oder Plasma (bspw. über den DAO-REA Test (Sciotec)), welche im Wesentlichen auf den Inhalten der Patente WO2007056788; EP 1948819 bzw. ihrer gemeinsamen Prioritätsunterlage AT 411688 beruht, diagnostische Aussagen zum Symptomkomplex der Histamin-Intoleranz zu treffen. Ein weiteres ELISA-basiertes Verfahren zur Bestimmung der DAO Aktivität im Blut ist aus Pfisterer et al., 2007 bekannt. Ein Verfahren zur Bestimmung der totalen Histaminabbaukapazität durch Differenzbildung einer gemessenen Probe zum Zeitpunkt t0min und t60min nach Inkubation bei 37°C, die aus einer gemeinsamen Ursprungsprobe stammen mit einem Radioimmunoassay ist aus Raithel et al., 1999 bekannt. Dabei erfolgt keine Zuführung einer externen Histaminlösung in eine der beiden Proben (t0 und t60) und damit keine Unabhängigkeit von der Probenmatrix. So zeigen die klinischen Erfahrungen der letzten Jahre allerdings, dass diese Messdaten nur mit geringer Signifikanz mit den klinischen Symptomen in Einklang zu bringen sind, was für den diagnostischen Einsatz der Systeme entsprechend nachteilig ist. So reagiert der DAO-REA Test und der Test nach Raithel et al., 1999 nur, wenn der Patient Symptome hat, er darf sich folglich nicht histaminfrei ernähren.

Praktisch wurde auch versucht insbesondere die Menge der DAO im Blut als Diagnose-Faktor heranzuziehen und zu etablieren, allerdings mit mäßigem Erfolg, insbesondere, weil die Symptome nicht von der Menge des Enzyms abhängen.

Vor diesem Hintergrund der Nachteile im Stand der Technik haben sich die Erfinder der vorliegenden Erfindung die Aufgabe gestellt, ein Verfahren zu entwickeln, das die im Stand der Technik genannten Nachteile wenigstens teilweise überwindet. Insbesondere haben sie sich zur Aufgabe gestellt, ein Verfahren zur Verfügung zu stellen, mit dem erstmals die totale Kapazität des Abbaus von Aminen, in einer biologischen Matrix (Probe) unabhängig von der Art des Abbauweges bzw. des oder der vorhandenen abbauenden Mediatoren, insbesondere von Histamin-abbauenden Enzymen und zusätzlich unabhängig vom Patienten-spezifischen Ernährungsstatus zu bestimmen ist.

Die Erfindung stellt hierzu ein Verfahren nach Anspruch 1 bereit. Bevorzugte Ausführungsformen sind in den Unteransprüchen ausgeführt.

Das erfinderische Verfahren zur Bestimmung der Amine-Abbaukapazität wird erläutert am Beispiel der totalen Histamin-Abbaukapazität in einer flüssigen biologischen Probe und umfasst dabei folgende Schritte:
- Aufteilen einer bereitgestellten Menge an flüssiger biologischer Probe zu gleichen Teilen in eine erste Teilprobe und eine zweite Teilprobe;
- Zugabe und Vermischen der ersten Teilprobe mit einer bestimmten Menge Histamin-Provokationslösung enthaltend Histamin;
- Inkubieren der mit Histamin-Provokationslösung versetzten ersten Teilprobe für einen bestimmten Zeitraum und unter bestimmten ersten Inkubationsbedingen bei denen das Histamin von etwaig in der Probe vorhandenen Mediatoren abgebaut werden kann, während zeitgleich die zweite Teilprobe für denselben bestimmten Zeitraum wie die erste Teilprobe und bei bestimmten zweiten Inkubationsbedingungen inkubiert wird, bei denen das Histamin von etwaig in der Probe vorhandenen Mediatoren nicht bzw. mengenmäßig nur unwesentlich abgebaut wird;
- Zugabe und Vermischen der zweiten Teilprobe nach besagter Inkubation mit derselben bestimmten Menge an Histamin-Provokationslösung, wie sie zuvor bei der ersten Teilprobe eingesetzt wurde;
- Entnahme einer mengengleichen Fraktion aus den Ansätzen der ersten und der zweiten Teilproben enthaltend Provokationslösung und Modifizieren des in beiden Ansätzen noch vorhandenen Histamins;
- Bestimmen der absoluten Konzentration des modifizierten Histamins (Histaminwert) in den beiden Teilproben;
- Bestimmen der (prozentualen) totalen Histamin-Abbaukapazität.

Die hier beschriebene Methode umgeht vorteilhaft alle vorweg beschriebenen Probleme im Stand der Technik, indem jede Messprobe vor der Messung in eine erste und zweite Teilprobe geteilt wird. Dem einen Teil der Gesamtprobe (erste Teilprobe) wird nun (sofort) eine definierte Menge Histamin-Provokationslösung zugesetzt. Sodann werden beide Teilproben einem zeitgleichen Inkubationsschritt, d.h. Inkubation für einen gleichen Zeitraum unterworfen, jedoch verschiedenen Inkubationsbedingungen, insbesondere anderen Temperaturprofilen unterzogen. Hieran anschließend wird auch dem anderen Teil der Gesamtprobe (zweite Teilprobe) die identische definierte Menge an Histamin-Provokationslösung zugesetzt und unmittelbar danach die Menge an Histamin in beiden Teilproben bestimmt.

Die Differenz der Messwerte spiegelt die totale Histamin-Abbaukapazität der Probe unter diesen Bedingungen wieder. In anderen Worten ausgedrückt, dient die zweite Teilprobe enthaltend Histamin-Provokationslösung als probeninterner Ausgangs-/ Basalwert (Histamin Eigenanteil der Probe und Histaminmenge der definierten Provokationslösung enthaltend Histamin) der Gesamtprobe, also als probeninterner Standard. Somit werden vorteilhaft alle probespezifischen Komponenten in die Messung mit einbezogen, so dass die Bestimmung der totalen Histamin-Abbaukapazität im Unterschied zum Stand der Technik sehr genau und weniger anfällig für Störungsgrößen erfolgen kann.

Weiterhin erfordert das erfindungsgemäße Verfahren keine äußere Histaminzufuhr bspw. durch die Nahrung oder Histaminprovokation beim Patienten. Der Patient kann sich sogar Histamin - frei ernähren und muss keine akuten Symptome nachweisen, was allein für das Befinden, insbesondere das allgemeine Wohlbefinden des Patienten vorteilhaft ist. Es erfolgt im erfindungsgemäßen Verfahren eine *in-vitro* Provokation der untersuchten flüssigen biologischen Probe, bspw. einer Serumprobe.

Das erfindungsgemäße Verfahren liefert somit einen zuverlässigen Hinweis auf eine mögliche Histamin-Intoleranz beim Patienten und somit vorteilhaft genauere klinische Rückschlüsse als dies mit Stand der Technik Verfahren aktuell möglich ist.

Mit dem erfindungsgemäßen Verfahren können vorteilhaft absolute und relative Histaminkonzentrationswerte genau bestimmt werden. Zusätzlich kann der prozentuale Histamin-Abbau, die totale Histamin-Abbaukapazität errechnet werden.

Ohne an die Theorie gebunden zu sein, wird angenommen, dass die Histamin-abbauenden Mediatoren insbesondere Diaminoxidase (DAO) und/oder Histamin-N-Methyltransferase (NMT) darstellen und/oder umfassen.

Die Wortfügung "Inkubationsbedingungen, bei denen das Histamin von etwaig in der Probe vorhandenen Mediatoren nicht bzw. mengenmäßig nur unwesentlich abgebaut wird" beschreibt im Rahmen dieser Erfindung solche Inkubationsbedingungen, die die Histamin-Abbau- bzw. - Umsetzungs-Aktivität von in der Probe befindlichen Mediatoren, wie bspw. von Enzymen, wie DAO und NMT entweder komplett in ihrer Aktivität behindern oder derart signifikant reduzieren, dass sie mit gängigen Methoden nicht nachweisbar ist oder nur derart unwesentlich hoch ist, dass vorhandenes Histamin nicht effizient über den angelegten bestimmten Zeitraum umgesetzt wird, wie bspw. eine Aktivität von ca. < 3U/ml.

Gemäß einer weiteren Ausführungsform des erfindungsgemäßen Verfahrens ist der bestimmte Zeitraum der Inkubation der ersten und der zweiten Teilprobe 1 bis 50 Stunden oder 5 bis 50 Stunden, bevorzugt 12 bis 36 Stunden, besonders bevorzugt 20 bis 28 Stunden.

Weiterhin umfassen die bestimmten ersten Inkubationsbedingungen eine Temperatur von 27°C bis 42°C, bevorzugt von 30°C bis 40°C, besonders bevorzugt von 34°C bis 38°C, und die bestimmten zweiten Inkubationsbedingungen der zweite Teilprobe umfassen demgegenüber eine Temperatur von 2°C bis 27°C, bevorzugt von 3°C bis 10°C, weiter besonders bevorzugt von 4°C bis 8°C.

Bei solchen angelegten Temperaturen wie bei der Inkubation der ersten Teilprobe ist es etwaig in der Probe vorhandenen Mediatoren möglich Histamin effektiv abzubauen bzw. umzusetzen, insbesondere die Histamin-Abbau- bzw. -Umsetzungs-Aktivität von in der Probe befindlichen Mediatoren, wie bspw. von Enzymen, wie DAO und NMT wird damit gefördert bzw. optimiert.

Im Vergleich werden bei solchen angelegten Temperaturen wie bei der Inkubation der zweiten Teilprobe der Abbau bzw. die Umsetzung von Histamin verhindert oder signifikant reduziert insbesondere durch einen inhibitorischen Einfluss auf die Histamin-Abbau- bzw. -Umsetzungs-Aktivität der Mediatoren, wie bspw. von Enzymen, wie DAO und NMT.

Alternativ liegt es auch im Rahmen der vorliegenden Erfindung, dass die zweiten Inkubationsbedingungen identisch sind zu den oben beschriebenen ersten Inkubationsbedingungen.

Gemäß einer bevorzugten Ausführungsform wird die totale Histamin-Abbaukapazität mit folgender Formel bestimmt: [(R - H) / (R / 100)], wobei R = Konzentration des modifizierten Histamins (Histaminwert) der zweiten Teilprobe (Referenzwert); H = Konzentration des modifizierten Histamins (Histaminwert) der ersten Teilprobe ist.

Erfindungsgemäß umfassen biologischen Proben unter Anderem aber nicht ausschließlich: Vollblut, Serum, Plasma, Cerebrospinalflüssigkeit, Saliva, Tränenflüssigkeit, Urin und/oder ein Homogenatvon z.B. einem Bioptat (Gewebeprobe) oder einer Stuhlprobe.

Bevorzugt ist die biologische Probe ausgewählt aus Vollblut, Serum oder Plasma.

Daneben können als biologische Matrix auch andere als die vorgenannten als biologische Probe in Betracht kommen. Insbesondere muss die untersuchte Probe nicht von sich aus flüssig sein, sondern kann mit geeigneten Mitteln und Verfahren flüssig gemacht (verflüssigt) werden, bzw. in Flüssigkeit aufgenommen werden.

Die biologische Probe kann von verschiedenen Spezies stammen, insbesondere von Spezies der Gruppe Säugetieren, darunter bspw. Mensch, Orang-Utan, Schimpansen, Maus, Ratte, Hund, Kaninchen, Ziege, Schaf, Rind und dgl..

Nach einer bevorzugten Ausführungsform stammt die biologische Probe vom Menschen.

Gemäß einer weiteren Ausführungsform enthält die Histamin-Provokationslösung Histamin in einem stabilisierenden Puffer in einer Konzentration zwischen 1 und 300 oder zwischen 1 und 100, bevorzugt zwischen 3 und 60 ng/ml oder zwischen 6 und 30 ng/ml und besonders bevorzugt zwischen 17 und 23 ng/ml. Geeignete Histamin stabilisierende Puffer sind dem Fachmann bekannt und sind hiermit vollumfänglich miteingeschlossen.

Bei der Bestimmung der Histaminkonzentration im erfindungsgemäßen Verfahren ist es unter Umständen erforderlich, das Histamin chemisch zu verändern, also zu modifizieren, so dass es von einem Antikörper erkannt werden kann. Histamin *per se* ist zu klein, um als Antigen zu fungieren und andererseits in jedem Säugetier ubiquitär vorhanden, sodass eine Immunisierung mit nicht modifiziertem Histamin prinzipiell zum Scheitern verurteilt wäre.

Die für eine immunologische Quantifizierung nötige Modifikation des Histamins wird z.B. als Acylierung durchgeführt. Jedoch sind dem Fachmann auch verschiedene andere Modifikationsmöglichkeiten bekannt und die erfindungsgemäße Methode ist nicht auf eine bestimmte und alleinige Modifikationsart zu beschränken.

Gemäß einer bevorzugten Ausführungsform umfasst der Verfahrensschritt der Modifikation des vorhandenen Histamins ein Acylieren.

Gemäß dem erfindungsgemäßen Verfahren kann die Konzentration an modifiziertem Histamin mittels Immunoassay, insbesondere Enzym-basierte Immunadsorptionsassays, wie insbesondere Enzyme-linked Immunosorbent assay (ELISA) und/oder Radioimmunoassay (RIA); Chromatographie, insbesondere mit Hoch-Leistungs-Flüssig-Chromatographie (HPLC); Gaschromatographie (GS); Elektrophorese oder Kapillarelektrophorese (CE)bestimmt werden.

Gemäß einer bevorzugten Ausführungsform wird die Konzentration an modifizierten Histamin mittels kompetitiven Enzyme-linked Imunosorbent assay bestimmt.

Gemäß einer weiteren bevorzugten Ausführungsform wird die Konzentration an modifizierten Histamin mittels Hoch-Leistungs-Flüssig-Chromatographie (HPLC) und/oder Gaschromatographie (GS) bestimmt.

In einer alternativen Ausführungsform des erfindungsgemäßen Verfahrens kann die Konzentration von Histamin auch direkt, d.h. ohne vorherige Modifikation von Histamin, z.B. mittels Chromatographie, insbesondere mit Hoch-Leistungs-Flüssig-Chromatographie (HPLC); Gaschromatographie (GS); Elektrophorese oder Kapillarelektrophorese (CE) bestimmt werden.

Die Konzentration an modifizierten und/oder unmodifizierten Histamin mittels Hoch-Leistungs-Flüssig-Chromatographie (HPLC) und Gaschromatographie (GS) kann bspw. dergestalt sein, dass zunächst eine Hoch-Leistungs-Flüssig-Chromatographie (HPLC) erfolgt, die den Erhalt wenigstens einer Fraktion (Fraktionierung) einer Eingangsprobe aufweist und anschließend die wenigstens eine Fraktion mittels Gaschromatographie (GS) weiter analysiert wird, d.h. methodisch eine HPLC-GS Kopplung vorliegt.

Das vorliegende Verfahren ist insbesondere interessant und vorteilhaft um Patienten, welche ernährungsbedingte Histamin assoziierte Symptome wie z.B. Verdauungsprobleme und/oder Hautirritationen haben, Klarheit über deren Ursachen zu verschaffen und ggf. entsprechende therapeutische Maßnahmen zu deren Linderung oder Abschaltung einzuleiten bzw. die Effizienz der eingeleiteten therapeutischen Maßnahmen zu überprüfen.

Aufgrund der bisherigen Datenlage wurde zur Diagnose von HIT die Bestimmung der Aktivität der Diaminoxidase (DAO)und die Bestimmung der Menge der DAO in Kombination mit einer ausführlichen Anamnese eingesetzt, dennoch konnten viele Betroffene nur unzureichend diagnostiziert werden, da die subjektive Symptomatik nur unzureichend mit den Messdaten aus den Verfahren des Standes der Technik korreliert.

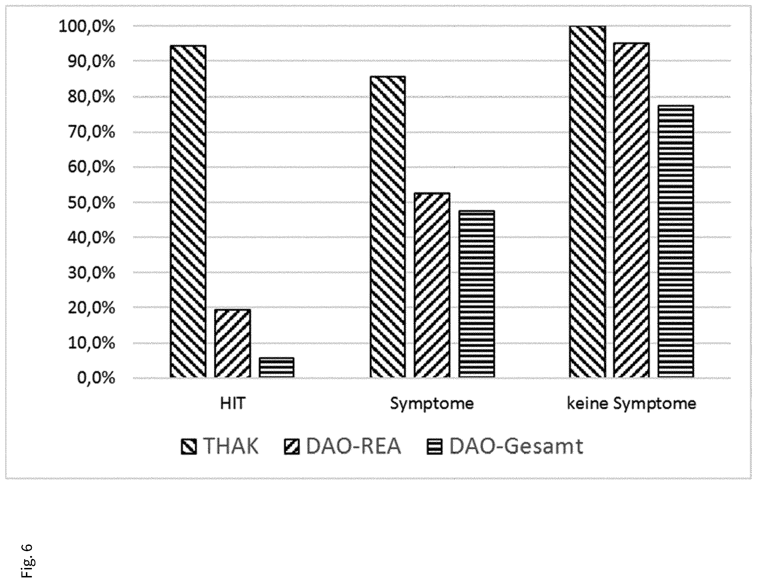

Durch das erfindungsgemäße Verfahren, dass eine interne Standardisierung einschließt, werden alle probespezifischen Komponenten in die Messung mit einbezogen und ermöglichen so erstmals eine zielsichere und klinisch relevante Diagnose (siehe Fig. 6).

So konnte ermittelt werden, dass Patienten, die eine totale Histamin-Abbaukapazität > 40% erreichen keine erkennbaren HIT Symptome zeigen. Ebenso konnte gezeigt werden, dass Patienten, die eine totale Histamin-Abbaukapazität < 25% zeigen, an deutlich erkennbaren HIT Symptomen leiden.

Insofern bietet die vorliegende Erfindung erhebliche Vorteile als Diagnose-Methode gegenüber solchen aus dem gegenwärtigen Stand der Technik, die bestimmte Patienten-Gruppen nicht richtig klinisch als unter einem defizienten Histaminabbau Leidende diagnostizieren können. Auch wird der Anteil an falsch negativ oder positiv klassifizierten Patienten mittels dem erfindungsgemäßen Verfahren reduziert.

Gemäß einer weiteren Ausführungsform wird die Bestimmung der totalen Histamin-Abbaukapazität als Grundlage für die Diagnose und/oder als Überwachung (Monitoring) einer therapeutischen Behandlung eines Krankheitsbildes eingesetzt, das auf einer defizitären totalen Histamin-Abbaukapazität basiert.

Ein solches typisches Krankheitsbild, bzw. ein typischer Symptomkomplex ist hierbei die sogenannte Histamin-Intoleranz (HIT) - wie oben beschrieben-, bei der der Körper mit vielgestaltigen Unverträglichkeitsreaktionen auf eine erhöhte Menge an Histamin reagiert.

Gleichzeitig ist das vorliegende Verfahren eine erstmalige vorteilhafte Gelegenheit um Anästhesierisiken, die durch eine überschießende Histaminausschüttung gepaart mit einer defizitären totalen Histamin-Abbaukapazität bedingt sind, zu kategorisieren und nachfolgend ausschließen bzw. ein entsprechendes Risiko einschätzen und bewerten zu können.

Dem entsprechend wird gemäß einer weiteren Ausführungsform das erfindungsgemäße Verfahren zur Bestimmung der totalen Histamin-Abbaukapazität als Grundlage für die Risikoeinschätzung im Vorfeld einer Anästhesie bei einem Krankheitsbild eingesetzt, welches auf einer defizitären totalen Histamin-Abbaukapazität basiert.

Es hat sich weiterhin gezeigt, dass in manchen Teilproben die dort vorliegende Diaminooxidase z.B. von Wasserstoffperoxid, einem ihrer Reaktionsprodukte, inhibiert werden kann. Zur Verhinderung bzw. zur Reduktion dieser Inhibition, wird - gemäß einer weiteren Ausführungsform - zu der Gesamtprobe vor Ihrer Teilung in eine erste und eine zweite Teilprobe und/oder nach derTeilung zur ersten und zweiten Teilprobe Peroxidase (EC 1.11.1), vorzugsweise Katalase (EC 1.11.1.6), zugesetzt.

Gemäß einer bevorzugten Ausführungsform wird die Peroxidase bzw. Katalase in einer Menge von 0,1 bis 10, vorzugsweise von 0,2 bis 5, noch mehr bevorzugt von 0,5 bis 2 Einheiten, insbesondere von 1 Einheit pro 100µl Probe zugesetzt.

Ein weiterer Aspekt der vorliegenden Anmeldung betrifft ein Kit und ein weiterer Aspekt der vorliegenden Erfindung betrifft die Verwendung dieses Kits zur Bestimmung der totalen Histamin-Abbaukapazität in einer flüssigen biologischen Probe nach dem erfindungsgemäßen Verfahren.

Dabei umfasst das Kit zumindest:
- Ein oder mehrere Probenaufnahmegefäße
- eine Histamin-Provokationslösung enthaltend Histamin in einem stabilisierenden Puffer in einer Konzentration zwischen 1 und 300 ng/ml oder zwischen 1 und 100 ng/ml, bevorzugt zwischen 3 und 60 ng/ml oder zwischen 6 und 30 ng/ml und besonders bevorzugt zwischen 17 und 23 ng/ml;
- ein oder mehrere Probenaufnahmegefäße optional enthaltend eine Platte beschichtet mit modifiziertem Histamin, insbesondere acyliertem Histamin;
- ein Histamin-Modifikationsreagens, insbesondere ein Histamin-Acylierungsreagens;
- Mittel zur Bestimmung von modifiziertem Histamin, insbesondere ELISA-basiert;
- optional eine Peroxidase oder Katalase, vorzugsweise in lyophylisierter Form;
- optional ein Histamin-Modifikationspuffer, insbesondere ein Histamin-Acylierungspuffer und
- optional ein modifizierten Histamin-Standard, insbesondere acyliertes Histamin.

Dabei umfasst das Histamin-Modifikationsreagens im Wesentlichen die für die Modifikation eines Histamins erforderlichen Chemikalien, Puffer und dergleichen. Als wichtigster und gegebenenfalls einziger Bestandteil des Histamin-Modifikationsreagens ist oder sind jene Reagentien anzusehen, die für die Modifikation des Histamins verantwortlich sind (z.B. ein Acylgruppendonor).

Ferner kann das Kit Mittel zur Bestimmung von modifiziertem Histamin enthalten. Diese Mittel umfassen vorzugsweise Antikörper gerichtet gegen modifiziertes Histamin, insbesondere polyklonale Antikörper, und gegebenenfalls sekundäre Antikörper, wobei mindestens einer dieser Antikörper vorzugsweise enzymatisch oder chemisch markiert ist. Daneben können diese Mittel bspw. auch Aptamere und andere, dem jeweiligen Stand der Technik entsprechende bioaffine Reagenzien umfassen. Die dabei vorgesehenen Antikörper können je nach Verwendung von verschiedensten Quellen stammen (z.B. von einer Zellkultur, von einem Tier; wie Schaf, Kaninchen oder Maus) und markiert (z.B. mit einem Enzym, mit Biotin, mit Streptavidin, mit einem Radionuklid) oder unmarkiert sein. Die Verfahren können auf Messung der Lichtabsorption oder der Transmission bzw. durchdringenden Lichtintensität bspw. mittels Turbidimetrie beruhen.

Insbesondere sind diese Mittel ELISA-basiert. Bei einem insbesondere ELISA-basiertem Mittel kann das Kit optional zumindest noch einen der nachfolgenden Bestandteile zusätzlich aufweisen:
- ein Antiserum gegen modifiziertes, insbesondere acyliertes Histamin
- ein Enzym-gekoppeltes Konjugat
- ein Substrat für das Enzym des Enzym-gekoppelten Konjugats
- ein Waschpuffer
- ein Stopppuffer zum Abstoppen einer Enzym-gekoppelten Farbstoffumsatzreaktion.

Zur Durchführung einer positiven Kontrollreaktion, bei der eine definierte Menge an DAO und/oder NMT eingesetzt werden kann, kann das Kit optional auch ein stabilisiertes DAO- und/oder NMT-Präparat enthalten. Dieses DAO/NMT-Präparat kann beispielsweise lyophilisiertes oder kältestabilisiertes DAO/NMT enthalten.

Ferner kann das Set auch optional einen Histamin-Modifikationspuffer, insbesondere ein Histamin-Acylierungspuffer umfassen, welcher sich insbesondere zur Modifikation/Acylierung von Histamin eignet. Der Histamin-Modifikationspuffer kann im Kit auch in konzentrierter Form vorliegen.

Optional kann das Kit auch modifiziertes, insbesondere acyliertes Histamin enthalten, welches als Standard bei der Bestimmung der totalen Histamin-Abbaukapazität in einer flüssigen biologischen Probe, insbesondere über die Bestimmung der Menge an modifizierten Histamin, herangezogen werden kann.

Gemäß weiterer Ausführungsformen, ist das erfinderische Verfahren anpassbar und einsetzbar um die Abbaukapazität andere biogene Amine zu bestimmen.

### Referenzen

### Morel et al.. 1988:

A M Morel; M A Delaage: "Immunoanalysis of histamine through a novel chemical derivatization",
J. ALLERGY& CLIN. IMMUNOL., Bd. 82, Nr. 4, 1. Oktober 1988, Seiten 646-654.

### Pfisterer et al. 2007:

M Pfisterer; A Missbichler: "New diagnostic and treatment opportunities of food-intolerance caused by histamine: first clinical results", ALL, Bd. 62, Nr. Suppl. 83, 1. Juni 2007, Seite 333.

### Raithel et al.. 1999:

M Raithel; M Küfner; P Ulrich; EG Hahn: "The involvement of the histamine degradation pathway by diamineoxidase in manifest gastrointestinal allergies", Inflamm. Res. 48, Supplement 1, (1999) S75-S76.

Die Erfindung wird im Weiteren durch nachfolgende Figuren und Beispiele erläutert, ohne jedoch auf diese beschränkt zu sein.

### Kurze Beschreibung der Figuren:

**Figur 1****:** Nachweis, dass Histamin bei weitem nicht das häufigste biogene Amin in Nahrungsmitteln ist. Dargestellt ist in mg/Portion Nahrungsmittel (Getränk oder Speise) die Menge an verschiedenen biogenen Aminen darunter von Histamin in Getränken und Speisen. Bei den dargestellten biogenen Aminen ist das Amin Cadaverin bei Speisen signifikant bei Weitem am höchsten mengenmäßig vertreten, gefolgt von den Aminen Putrescin und Tyramin. Demgegenüber ist im Vergleich Histamin in Speisen mengenmäßig etwa im Mittelfeld bei den untersuchten Aminen angesiedelt. In Getränken stellt Histamin jedoch die signifikant höchste Menge im Vergleich zu den anderen untersuchten Aminen, jedoch ist die Gesamtmenge/Portion recht gering im Vergleich zur Menge an Cadaverin und Putrescin in Speisen.
**Figur 2****:** Konzentrationskurve der Verdünnungsreihe des acylierten Histaminstandards des ELISA der vorliegenden Erfindung (Standardkurve) bei einer Absorption von 450 nm.

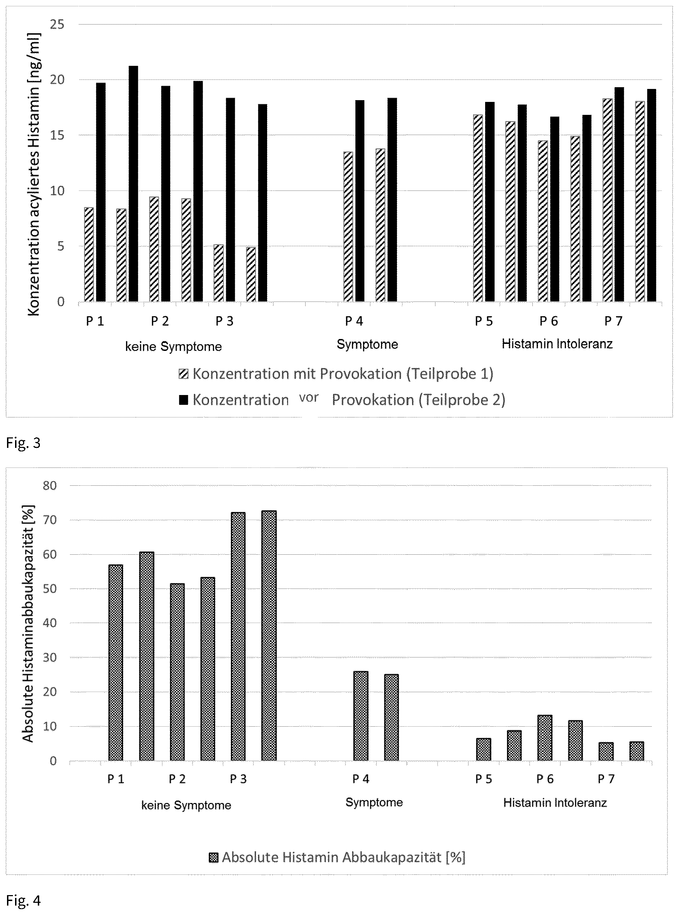

**Figur 3/4****:** Dargestellt ist eine typische Konzentrationsverteilung der beiden Teilproben (erste und zweite Teilprobe), wobei die Patientengruppen nach der vorhandenen klinischen Bewertung gebildet wurden. Die ersten drei Patienten hatten keine Symptome (P1-3). Patient 4 (P4) hatte Symptome und Patienten 5-7 (P5-7) eine Histamin-Intoleranz (HIT). Figur 3 zeigt die Konzentrationsverteilung der Teilproben jeweils vor und nach *in-vitro* Histamin-Provokation in Doppelbestimmung pro Patient. Figur 4 zeigt die prozentuale totale Histamin-Abbaukapazität der Proben errechnet anhand der Konzentrationen aus Fig. 3. **Rechts:** Patienten mit diagnostizierter HIT, geringe/unzureichende totale Histamin-Abbaukapazität (Ein Abbau im Bereich von 0 - 25% kennzeichnet eine insuffiziente oder pathologisch niedrige Histamin-Abbaukapazität; pathologisch); **Mitte:** Patienten mit Symptomen aber HIT nicht diagnostizierbar (Ein Abbau im Bereich von 25 - 40% kennzeichnet eine eingeschränkte totale Histamin-Abbaukapazität; grenzwertig); **Links:** Gesunde Probanden, keine Symptome (Ein Abbau im Bereich von > 40% kennzeichnet eine ausreichende totale Histamin-Abbaukapazität, (HIT) negativ).

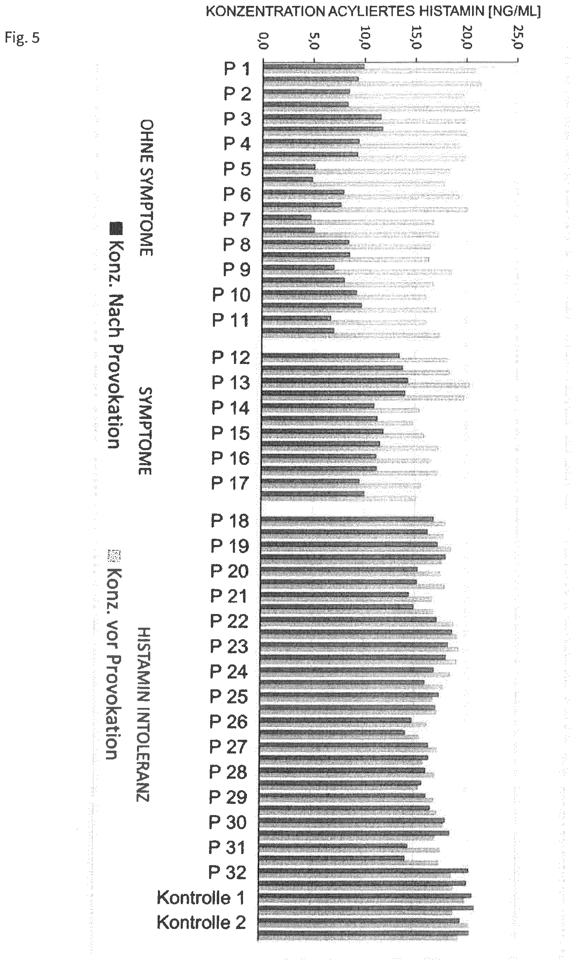

**Figur 5****:** Zeigt eine typische Konzentrationsverteilung mit einem größeren Patientenkollektiv, Gesamtprobandenzahl: n= 32, sowie zwei Kontrollen die keinen signifikanten Histamin-Abbau aufweisen dürfen. Die Einteilung der Patienten findet wie in Fig. 4 in drei Patienten-Gruppen anhand der totalen Histamin-Abbaukapazität gemessen in ihren Proben nach dem erfindungsgemäßen Verfahren statt.
**Figur 6****:** Dargestellt ist auf der y-Achse die prozentuale Spezifität des erfindungsgemäßen Verfahrens zur Klinik im Vergleich zum DAO-REA Test (Sciotec) bzw. DAO-Gesamt ELISA (Immundiagnostik). Getestet wurde ein Patientenkollektiv mit 119 Patienten, die entweder keine Symptome hatten (Balken: rechts), Symptome, die auf eine Histamin Unverträglichkeit hinweisen (Balken: Mitte) oder eine diagnostizierte HIT haben (Balken: links).

Das vorliegende erfindungsgemäße Verfahren kann insbesondere als diagnostischer Test eingesetzt werden und ist inzwischen ggf. mit einigen Abwandlungen als Frost Diagnostika Totale Histamin-Abbaukapazität Test, kurz (FD THAK / THDC Test) im Handel verfügbar. Das erfindungsgemäße Testsystem beruht hierbei auf einem Enzyme Linked Immunosorbent Assay (ELISA) zum quantitativen Nachweis der totalen Histamin-Abbaukapazität in der Serumprobe. Vorgesehen ist dieser Test in erster Linie zur *in*-*vitro*-diagnostischen Anwendung in Krankenhäusern oder medizinischen Fachlabors. Andere Anwendungsgebiete sind gleichwohl denkbar und gewünscht.

Der Test bestimmt erstmals die Abbaukapazität von Histamin in einer Serumprobe, unabhängig von der Art des Histamin-Abbaus. Andere flüssige und feste biologische Proben wie bspw. Vollblut oder Blutplasma, Stuhlproben, Bioptate sind dabei auch denkbar. Dies ist insofern von Bedeutung, da diese Abbaukapazität die Problematik der Histamin-Intoleranz darstellt. Da die basale Histamin-Konzentrationen in Serum Proben hohe Konzentrationsschwankungen zeigen, liefert das erfindungsgemäße Verfahren erstmalig einen zuverlässigen Hinweis und eine direkte Korrelation zwischen totaler Histamin-Abbaukapazität und dem Symptomkomplex Histamin-Intoleranz beim untersuchten Patienten.

Die Durchführung des Tests verlangt vorteilhafter Weise keine Histamin Zufuhr oder Histamin Provokation beim Patienten, welche immer mit entsprechenden Nachteilen beim Patienten verbunden ist. Der Patient kann sich sogar Histamin-frei ernähren und muss keine akuten Symptome nachweisen, denn es erfolgt im erfindungsgemäßen Verfahren eine *In-vitro* Histamin-Provokation der Serumprobe mittels einer applizierten Histamin-Provokationslösung und somit wird die totale Histamin-Abbaukapazität auch unabhängig von dem spezifischen Ernährungsstatus des Patienten bestimmt.

### Beispiel 1:

### Detaillierte Beschreibung des erfindungsgemäßen Test

Der FD THAK/ THDC ELISA Test gemäß der vorliegenden Erfindung basiert auf einer enzymatischen Farbreaktion und gehört somit zu den enzymatischen Immunadsorptionsverfahren (EIA). Der Test enthält Materialien um die totale Histamin-Abbaukapazität von Serumproben zu bestimmen.

Der entwickelte ELISA-basierte Enzymimmunoassay (kompetitive Assay) zur Messung der totalen Histamin-Abbaukapazität in biologischen Proben zeichnet sich durch die Unabhängigkeit von der Probenmatrix aus. Da jede Probe gegen sich selbst als Referenzwert gemessen wird, werden alle denkmöglichen Einflüsse der Probenmatrix systemintern neutralisiert. Vorteilhaft werden somit probeninterne Messschwankungen eliminiert.

Neben dem hier beschriebenen kompetitiven ELISA Assay sind auch andere ELISA Verfahren, bspw. der klassische Sandwich-ELISA gemäß dem erfindungsgemäßen Verfahren möglich.

Die Serumproben werden geteilt (Proben A und B) und in Einfach-Bestimmung in eine Inkubationsplatte pipettiert (Probenreihe A). Anschließend wird eine Histamin-Provokationslösung zu den ersten Teilproben (Probenreihe A) zugegeben. Danach wird die Inkubationsplatte mit der ersten Teilprobe (Probenreihe A) für 24 h bei 37 °C inkubiert.

Nach 24 h wird die Serumprobe der zweiten Teilprobe in Probereihe B parallel zur Probenreihe A in die Inkubationsplatte pipettiert. Danach wird ebenfalls die gleiche Menge Histamin-Provokationslösung wie bei der ersten Teilprobe (Probenreihe A) auf die zweite Teilprobe (Probereihe B) gegeben (siehe Tab. 1).

Nach der Probenvorbereitung wird aus der Inkubationsplatte ein einfacher Acylierungschritt durchgeführt. Aus dieser Acylierung wird anschließend der Histamin-Abbaukapazitäts-ELISA durchgeführt.

Im Verlauf der Probenaufbereitung wird Histamin zu N-Azyl-Histamin modifiziert/acyliert. Der sich anschließende kompetitive ELISA basiert auf dem Mikrotiterplattenformat, gleichsam sind Abwandlungen möglich und dem Fachmann bekannt. Das Antigen (acyliertes Histamin) ist an eine feste Phase gebunden (mit acyliertem Histamin-beschichtete Mikrotiterplatten). Die Analytkonzentrationen der Standards, Kontrollen und Proben und die an der festen Phase gebundenen Analytkonzentrationen, konkurrieren um die vorhandenen Bindungsstellen der daraufhin zu- und eingesetzten gelösten Antikörper, die im Unterschuss vorhanden sind. Im Gleichgewicht werden die freien Antigene und die freien Antigen-Antiseren-Komplexe durch Waschen entfernt. Je weniger acyliertes Histamin in der Eingangsprobe vorhanden ist, desto mehr Antikörper können an acylierte Histamine (Antigene) der Plattenbeschichtung binden. Der an der festen Phase an acyliertes Histamin gebundene Antigen-Antikörper-Komplex wird mit einem enzymmarkierten Antikörper bestimmt und mit einem Substrat durch eine Farbreaktion nachgewiesen. Die Farbumsetzung wird bei Messung der Absorption bei 450 nm ermittelt. Die Konzentrationen in den Proben werden mit Hilfe einer Standardkurve aus Verdünnungen des Standards (siehe Fig. 2) vorteilhaft sehr genau ermittelt.

Durch die Messung mit dem Histamin-Abbaukapazitäts-ELISA kann anschließend vorteilhaft und genau die Konzentration der Probe vor Histamin-Provokation und mit Histamin-Provokation ermittelt werden. Vorteilhaft können also absolute und relative Histaminkonzentrationswerte genau bestimmt werden. Zusätzlich kann der prozentuale Histamin-Abbau, die totale Histamin-Abbaukapazität errechnet werden (siehe Fig.3/4).

Das erfindungsgemäße Verfahren bzw. die erfindungsgemäße Verwendung des Kits in besagten Verfahren lässt sich mit entsprechender Modifikation darüber hinaus prinzipiell auch zur Bestimmung der Abbaukapazität anderer medizinisch relevanter Amine einsetzen bspw. der eingangs genannten anderen Amine, die durch DAO und/oder NMT umgesetzt werden, wie Putrescin, Cadaverin, Spermin, Spermidin, Tyramin und Phenylethylamin.

### Beispiel 2:

In einem Testbeispiel wurde der Ansatz in geeigneten Polypropylen-Röhrchen oder DeepWell-Platten durchgeführt zum quantitativen Nachweis der totalen Histamin-Abbaukapazität in der jeweiligen Serumprobe.

Das allgemeine Procedere wird zunächst übersichtshalber dargestellt und dann im Detail.

### Kurzübersicht:

Übersichtshalber gestaltete es sich hierbei, wie folgt:
- Je 100 µl der Gesamtprobe wurden vorgelegt und mit 500µl Histamin-Provokationslösung in Reaktionsgefäß "H" (Histamin-Messprobe, erste Teilprobe) pipettiert
- Je 100 µl der Gesamtprobe wurden in Reaktionsgefäße "R" (Referenz) pipettiert (zweite Teilprobe)
- Reaktionsgefäße "H" wurden verschlossen und 24h bei 37°C inkubiert
- Reaktionsgefäße "R" wurden verschlossen und 24h bei 4°C oder alternativ 6°C gelagert
- In die Reaktionsgefäße "R" wurde 500µl Histamin-Provokationslösung pipettiert
- In die Reaktionsgefäße "M"(Modifikation) wurden je 50µl aus den Reaktionsgefäßansätzen "H" bzw. "R" pipettiert und dann 25µl Modifikationslösung und dann 250µl Reaktionspuffer hinzu pipettiert
- Inkubiert wurde daraufhin für 30 min bei Raumtemperatur (RT)
- sodann wurde die Konzentrationen von modifiziertem Histamin in allen Teilproben mit Hilfe einer Standardkurve (siehe Fig. 2) bestimmt
- Aus der Differenz der Konzentrationen von modifizierten Histamin [ R- minus H] errechnet sich die Abbaukapazität in absoluten Konzentrationen bzw. [ (R-H) / (R/100)] der prozentuelle Abbau.

Aufgrund des absolut neuartigen Ansatzes der internen Standardisierung mit der eigenen Probe setzt das vorliegende erfinderische Testsystem auch neue Maßstäbe bei der unterstützenden Diagnose einer so genannten Histamin-Intoleranz (HIT).

### Versuchsbeschreibung:

Vorliegend wurden Blutserumproben von gesunden Probanden (Patienten) und Patienten mit vermuteten Defiziten in der totalen Histamin-Abbaukapazität untersucht. Es wurde nurfrisches, blutplättchenarme, nicht inaktivierte Seren benutzt.

### Serumgewinnung:

- Zur Serumgewinnungwurde das Blut bis zur vollständigen Gerinnung in einem Röhrchen (ohne Zusatz) bei Raumtemperatur stehen gelassen. Die Zeit bis zum Eintritt der Gerinnung betrug 30 min bis zu 2 Stunden.
- Anschließend wurde das Material 5-10 Minuten bei 1000 - 3000 x g zentrifugiert und der Serumüberstand abpipettiert.

### Haltbarkeit und Behandlung der Seren:

- Serum - Verdünnungen wurden immer frisch angesetzt
- Seren wurden kühl gelagert (2 - 8°C)
- Verschlossene Probengefäße wurden verwendet
- Lagerung:
   ∘ max. 1 bis 3 Tage bei Raumtemperatur, 5 Tage bei 2 - 8°C
   ∘ mehrere Monate bei - 20 °C
   ∘ mehrere Jahre bei - 80 °C
- Mehrmaliges Auftauen wurde vermieden.

### Testdurchführung

### Probenvorbereitung

Die zu messende Serumprobe (Gesamtprobe) wurde gevortexed und jeweils in gleicher Menge in Aliquots aufgeteilt
→ Probe **A** und **B** (erste und zweite Teilprobe der Gesamtprobe)

### Inkubationsplatte

Die Aliquots der zu testenden frischen Serumproben (Gesamtproben) wurden jeweils in gleicher Menge in eine erste und eine zweite Teilprobe geteilt und in Einfach-Bestimmung in eine Inkubationsplatte pipettiert (erste Teilprobe: Probereihe A).
- Es wurden 100 µl Probe A (erste Teilprobe) wie in der Belegung in Tabelle 1 dargestellt pro Testperson in die Inkubationsplatte pipettiert.
- 500 µl Provokationslösung wurde daraufhin zu Proben A hinzugeben
- Die Inkubationsplatte wurde mit Silberfolie abgeklebt und auf einem Schüttler bei 37°C und 600 rpm für 24 h inkubiert.
- Die Probe B (zweite Teilprobe) wurde 24h bei 4-8°C gelagert
- Nach der Inkubation wurde die Folie abgezogen und die Platte sich für 10 min abkühlen gelassen
- Jeweils 100 µl Probe B laut Belegung (siehe Tabelle 1) wurden nach den 24 h in die Inkubationsplatte pipettiert
- Daraufhin wurden 500 µl Provokationslösungzu den jeweiligen Proben B hinzugegeben
- Die Inkubationsplatte wurde 1 min bei 600 rpm zum Mischen auf den Schüttler gestellt.

### Acylierungsplatte (siehe Belegungsplan nach Tabelle 2)

### Acylierung

1. Jeweils **50 µl** der **Standards, Kontrollen und Proben** wurden in die entsprechenden Kavitäten der **Acylierungsplatte** pipettiert. Die Verdünnungen des Standards (Standard A bis F) zur Generierung einer Standardkurve (siehe Figur 2) waren bereits gebrauchsfertig in separaten Reaktionsgefäßen vorhanden.
2. **25 µl Acylierungsreagenz** wurde in alle Kavitäten pipettiert
3. **250 µl Acylierungspuffer** wurde in alle Kavitäten pipettiert.
   Die Platte wurde nicht abgeklebt oder verschlossen.
4. **30 Min** wurde **bei Raumtemperatur (RT)** (20-25 °C) auf einem Schüttler (ca. 600 rpm) inkubiert.
5. Jeweils **25 µl** der vorbereiteten Standards, Kontrollen und Proben wurden für den ELISA benötigt

### Beispielbelegung

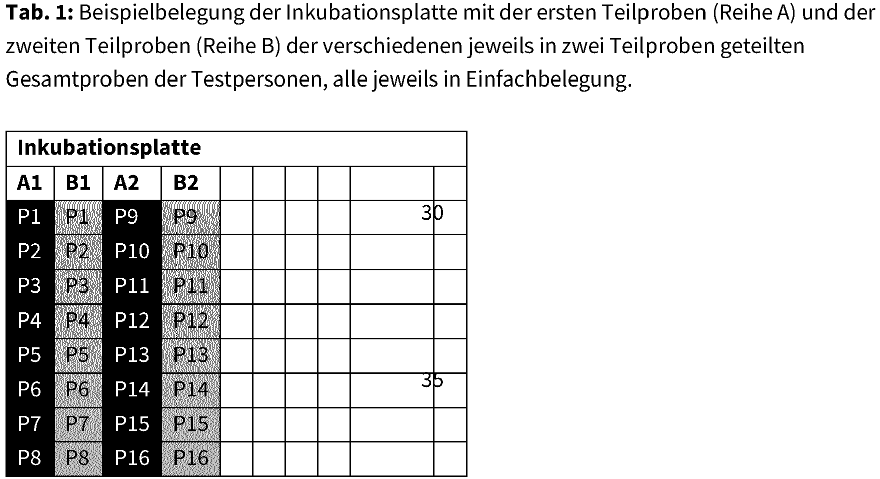

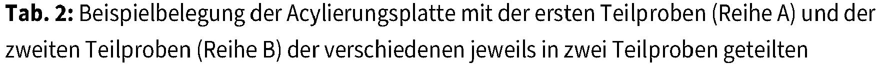

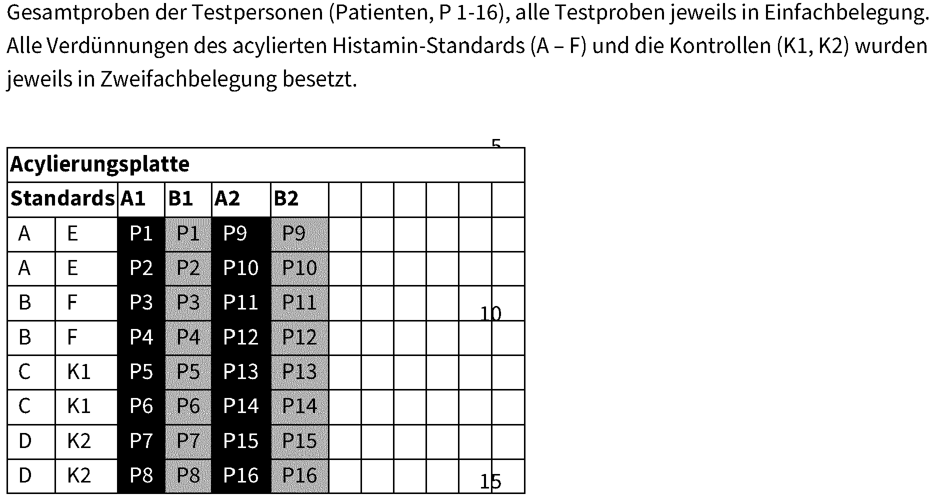

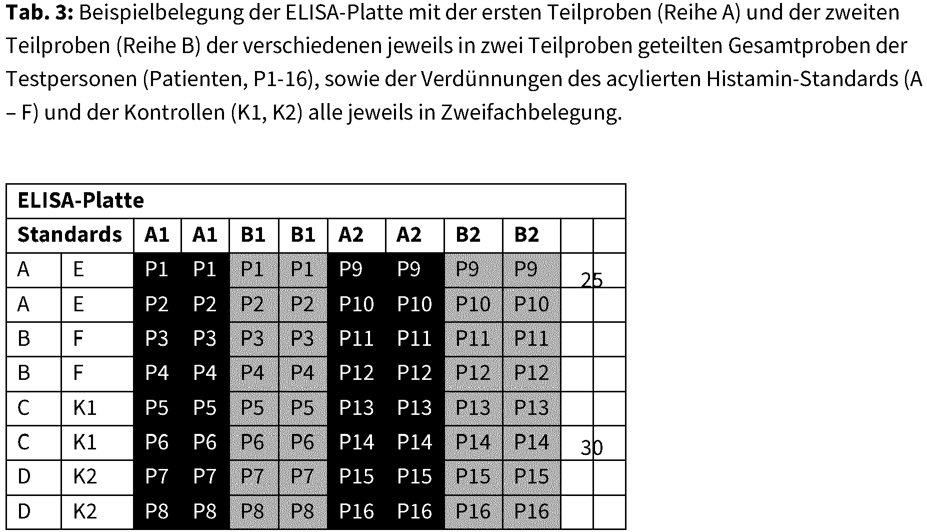

### ELISA-Platte

### ELISA

1. 25 µl der acylierten Standards, Kontrollen und Proben wurden in die entsprechenden Kavitäten der ELISA-Mikrotiterplatte pipettiert (siehe Belegungsplan nach Tabelle 3). Die ELISA-Platte ist hierbei insbesondere am Boden der Kavitäten mit Histamin vobeschichtet (kompetitiver ELISA).
2. **100 µl Histamin Antiserum** wurde daraufhin in alle Kavitäten hinzugeben.
3. Die Platte wurde für **30 min bei RT** (20 - 25 °C) auf einem **Schüttler (ca. 600 rpm)** inkubiert. Die Platte wurde mit einer Folie verschlossen.
4. Die Folie wurde entfernt und der Inhalt der Kavitäten wurde ausleert oder abgesaugt. Die Kavitäten wurden 3-mal gründlich mit 300 µl Waschpuffer (1x) gewaschen, dann ausgeleert und die Restflüssigkeit jedes Mal durch Ausklopfen auf einer saugfähigen Unterlage entfernt.
5. **100 µl Konjugat** wurde in alle Kavitäten pipettiert.
6. **15** min oder alternativ 20 min wurden die Proben daraufhin bei RT (20 - 25 °C) auf einem **Schüttler (ca. 600 rpm)** inkubiert.
7. Der Inhalt der Kavitäten wurde anschließend ausgeleert oder absaugt. Die Kavitäten wurden 3 x gründlich mit **300 µl** Waschpuffer gewaschen, ausgeleert und die Restflüssigkeit jedes Mal durch Ausklopfen auf einer saugfähigen Unterlage entfernt.
8. **100 µl** SUBSTRAT wurde in alle Kavitäten pipettiert und für 15 min bei RT (20 - 25 °C) auf einem Schüttler (ca. 600 rpm) inkubiert. Direktes Sonnenlicht wurde vermieden.
9. **100 µl** Stop Lösung wurde in alle Kavitäten pipettiert.
10. Die Absorption wurde mit einem Mikrotiterplatten-Reader bei 450 nm (gegen eine Referenzwellenlänge von 620-650 nm) innerhalb von 10 Minuten gemessen.

### Testauswertung

Durch die Zugabe der Provokationslösung entsteht eine 1:1,2 Verdünnung. Die aus der Standardkurve abgelesenen Konzentrationen (siehe Fig. 2) müssen und wurden daher mit dem Faktor 1,2 multipliziert um die tatsächliche Histamin Konzentration zu erhalten.

Bei der Auswertung wird die prozentuale Histaminabbaukapazität der gesamten Serumprobe errechnet. Hierzu werden beiden gemessenen Konzentrationen, sowohl von der mit Histamin-Provokationslösung inkubierten Probe (Probe A, erste Teilprobe) als auch von der Probe die frisch pipettiert wurde (Probe B, zweite Teilprobe), ins Verhältnis gesetzt.

% Histamin-Abbau oder totale Histamin-Abbaukapazität = [(R - H) / (R /100)], wobei R = Konzentration des acylierten (modifizierten) Histamins (Histaminwert) der zweiten Teilprobe (Probe B; Referenzwert); H= Konzentration des acylierten (modifizierten) Histamins (Histaminwert) der ersten Teilprobe (Probe A).

### Beispielrechnung

### Patient 1:

Probe A (erste Teilprobe) mit Histamin-Provokationslösung, 24 h inkubiert:
   9,913 [ng/ml]
Probe B (zweite Teilprobe) mit Histamin-Provokationslösung, die nach 24 h Inkubation pipettiert wurde, woran direkt die Messung anschloss:
   20,819 [ng/ml].

### Patient 2:

Probe A (erste Teilprobe) mit Histamin-Provokationslösung, 24 h inkubiert:
   11,579 [ng/ml]
Probe B (zweite Teilprobe) mit Histamin-Provokationslösung, die nach 24 h Inkubation pipettiert wurde, woran direkt die Messung anschloss:
   19,772 [ng/ml].

Für die Gesamtprobe des Patienten Nr. 1 gilt: (20,819 - 9,913) / (20,819 / 100) = 52,38%

Für die Gesamtprobe des Proband Nr. 2 gilt: (19,772 - 11,579) / (19,772 / 100) = 41,44 %

Das bedeutet Proband Nr. 1 hat eine totale Histamin-Abbaukapazität von 52,3%, Proband Nr. 2 hat demgegenüber eine totale Histamin-Abbaukapazität von 41,44 %.

### Interpretation der Ergebnisse, Bezug zur Klinik

Die resultierenden Ergebnisse können wie folgt interpretiert werden:
0 % - 25 %: **geringe bis keine totale Histamin-Abbaukapazität:** Die Probe des Patienten weist eine sehr niedrige totale Histamin-Abbaukapazität auf. Man kann hier vom Vorliegen einer Histamin-Intoleranz bei dem Patienten ausgehen.
25 % - 40 %: **eingeschränkte totale Histamin-Abbaukapazität:** Man kann hier von einer Einschränkung bzw. Beeinträchtigung der totalen Histamin-Abbaukapazität der Probe bzw. bei dem Patienten ausgehen.
> 40 % **ausreichende** totale **Histamin-Abbaukapazität:** Man kann hier von keiner Einschränkung der totalen Histamin-Abbaukapazität bei der Probe bzw. bei dem Patienten ausgehen, d.h. der Patient ist bzgl. der Histamin-Abbaukapazität klinisch unauffällig.

Somit hat Patient 1 mit einer prozentualen totalen Histamin-Abbaukapazität von 52,38% eine physiologisch ausreichende totale Histamin-Abbaukapazität. Es handelt sich um einen klinisch unauffälligen Probanden.

Patient 2 liegt mit einer prozentualen totalen Histamin-Abbaukapazität von 41,44 % knapp über der Schwelle einer physiologisch ausreichenden totalen Histamin-Abbaukapazität. Es handelt sich um einen klinisch unauffälligen Probanden im unteren unauffälligen Schwellenbereich. Eine weitere klinische Verfolgung der totalen Histamin-Abbaukapazität bei diesem Patienten ist hierbei ratsam.

### Leistungsmerkmale des Tests

### Präzision und Reproduzierbarkeit

### Intra-Assay Varianz (Präzision)

Die Intra-Assay Varianz wurde beispielhaft mit mehreren Messungen (n=39) zweier verschiedener Proben dargestellt und als hinreichend befunden. Probe 1 wies einen Mittelwert von 0,6 ng/ml und eine Standardabweichung von ± 0,1 ng/ml auf (12 % Variationskoeffizient (CV)). Probe 2 wies einen Mittelwert von 4,6 ng/ml und eine Standardabweichung von ± 0,3 ng/ml auf (6,3 % Variationskoeffizient (CV)).

### Inter-Assay Varianz (Präzision)

Die Inter-Assay Varianz wurde beispielhaft mit mehreren Messungen (n=13) zweier unterschiedlicher Proben an unterschiedlichen Tagen durch verschiedene Personen dargestellt und als hinreichend befunden. Probe 1 wies einen Mittelwert von 2,03 ng/ml und eine Standardabweichung von ± 0,16 ng/ml auf (8 % Variationskoeffizient (CV)). Probe 2 wies einen Mittelwert von 6,74 ng/ml und eine Standardabweichung von ± 0,37 ng/ml auf (5,6 % Variationskoeffizient (CV)).

Spezifität (Kreuzreaktion) des Tests ergab für folgende Testsubstanzen eine prozentuale Kreuzreaktion gegenüber Histamin von:
- 3-Methyl-Histamin:: 0,1 %
- Tyramin:: 0,01 %
- L-Phenylalanin:: < 0,001%
- L-Histidin:: < 0,001%
- L-Tyrosin:: < 0,001%
- Tryptamin:: < 0,001%
- 5-Hydroxy-Indol-Essigsäure:: < 0,001%
- Serotonin:: < 0,001%

Die analytische Sensitivität (Limit of Detection) für acyliertes Histamin (N-Azyl-Histamin) liegt im Mittel bei 0,2 ng/ml + 2 Standardabweichungen.

Für die Wiederfindung von acyliertem Histamin gilt ein Bereich von 0,74 bis 8,48 ng/ml, entsprechend einem prozentualen Bereich von 85-106% mit einem Mittelwert von 100%.

Für die Linearität gilt eine serielle Verdünnung bis 1:16 von acyliertem Histamin, entsprechend einem prozentualen Bereich von 92-120% mit einem Mittelwert von 103%.

Der Messbereich für acyliertes Histamin liegt bei 0,1 - ca. 40 ng/ml. Eine genauere Quantifizierung unter- und oberhalb dieses Bereichs ist mitunter schwierig.

In Figur 3 ist eine typische Konzentrationsverteilung der beiden Teilproben (erste und zweite Teilprobe; Fig. 3) bzw. der totalen Histamin-Abbaukapazität (Fig. 4) dargestellt, wobei die Patientengruppen nach der vorhandenen klinischen Bewertung gebildet wurden (siehe Einteilung oben "Bezug zur Klinik").

Zu sehen sind in Fig. 3 geringe Messschwankungen innerhalb der modifizierten Histamin-Konzentrationen in den beiden Teilproben (erste und zweite Teilprobe) in jeder Probe bei deren Doppelbestimmung/-messung. Die Schwankungen sind jedoch zu vernachlässigen, da sich am Befund nichts verändert.

Bei der Darstellung der aus Fig. 3 errechneten prozentualen totalen Histamin-Abbaukapazität gemäß Figur 4 sind diese Schwankungen zwischen den doppelbestimmten Proben erst recht nivelliert und vernachlässigbar. Dies zeigt insbesondere die hohe Genauigkeit der Verwendung der prozentualen totalen Histamin-Abbaukapazität einer Probe gemäß des erfindungsgemäßen Verfahrens. Probanden ohne Symptome zeigen einen deutlichen Unterschied zwischen den Teilproben, mit einerAbbaukapazität von deutlich über 40%. Personen, welche mitunter Symptome haben aber mit der Diagnose nach dem Stand der Technik als gesund eingestuft wurden, zeigen eine deutlich reduzierte totale Histamin-Abbaukapazität zwischen 25% und 40%. Patienten, die mit der Diagnose nach dem Stand der Technik als Histamin-intolerant eingestuft wurden zeigen allesamt eine sehr niedrige totale Histamin-Abbaukapazität von unter 25% (siehe Fig. 4).

In Fig. 5 ist in Bezug auf die Figuren 3 und 4 eine typische Konzentrationsverteilung mit einem größeren Patientenkollektiv gezeigt. Die Einteilung der Patienten findet wie in Fig. 3/4 in drei Patienten-Gruppen anhand der totalen Histamin-Abbaukapazität gemessen in ihren Proben nach dem erfindungsgemäßen Verfahren statt.

Figur 6 zeigt den Bezug des Tests gemäß dem erfindungsgemäßen Verfahren zur Klinik, d.h. zur richtigen Diagnosestellung einer mutmaßlichen HIT-Erkrankung. Zu sehen ist, dass das erfindungsgemäße Verfahren 94,4 % der HIT Patienten korrekt als HIT Patienten erkannt hat, wobei die Stand der Technik Verfahren, nämlich der DAO-REA Test hier sehr nachteilig nur 19,4 % und der DAO-Gesamt ELISA sogar nur 5,6% richtig diagnostiziert hat. Patienten die nur Symptome hatten, wurden mit einer eingeschränkten totalen Histamin-Abbaukapazität vom erfindungsgemäßen Verfahren mit 85,7% Spezifität gefunden, wobei die prozentuale Spezifität zur Klinik bei den Stand der Technik Verfahren nach dem DAO-REA Test 52,4 % und beim DAO-Gesamt ELISA bei 47,6% lag.

Patienten ohne Beschwerden, also ohne Symptome, die auf eine HIT hindeuten, konnten mittels des erfindungsgemäßen Verfahrens zu 100% richtig diagnostiziert werden. Mit den Stand der Technik Verfahren wurden signifikant schlechtere richtige klinische Zuordnungen erzielt: Mit dem DAO-REA wurden hier 95,2 % richtig gefunden und mit dem DAO-Gesamt ELISA sogar nur 77,4%.

## Patentansprüche

1. Verfahren zur Bestimmung der totalen Histamin-Abbaukapazität in einer flüssigen biologischen Probe **umfassend die Schritte:**
- Aufteilen einer bereitgestellten Menge an flüssiger biologischer Probe zu gleichen Teilen in eine erste Teilprobe und eine zweite Teilprobe;
- Zugabe und Vermischen der ersten Teilprobe mit einer bestimmten Menge Histamin-Provokationslösung enthaltend Histamin;
- Inkubieren der mit Histamin-Provokationslösung versetzten ersten Teilprobe für einen bestimmten Zeitraum und unter bestimmten ersten Inkubationsbedingungen, während zeitgleich die zweite Teilprobe für denselben bestimmten Zeitraum wie die erste Teilprobe und bei bestimmten zweiten Inkubationsbedingungen inkubiert wird;
- Zugabe und Vermischen der zweiten Teilprobe nach deren Inkubation mit derselben bestimmten Menge an Histamin-Provokationslösung enthaltend Histamin wie zuvor bei der ersten Teilprobe eingesetzt wurde;
- Entnahme einer mengengleichen Fraktion aus den Ansätzen der ersten und der zweiten Teilproben enthaltend Provokationslösung und Modifizieren des in den Ansätzen noch vorhandenen Histamins;
- Bestimmen der absoluten Konzentration des modifizierten Histamins (Histaminwert) in den beiden Teilproben;
- Berechnung der totalen Histamin-Abbaukapazität

2. Verfahren nach Anspruch 1, **dadurch gekennzeichnet, dass**
die totale Histamin-Abbaukapazität mit folgender Formel bestimmt wird: [(R - H) / (R / 100)], wobei R = Konzentration des modifizierten Histamins (Histaminwert) der zweiten Teilprobe (Referenzwert) und H = Konzentration des modifizierten Histamins (Histaminwert) der ersten Teilprobe ist.

3. Verfahren nach Anspruch 1 oder 2, **dadurch gekennzeichnet, dass**
die flüssige biologische Probe Vollblut, Blutserum, Blutplasma, Cerebrospinalflüssigkeit, Saliva Urin, und/oder ein Homogenat eines Bioptats (Gewebeprobe) oder einer Stuhlprobe ist.

4. Verfahren nach einem der vorangehenden Ansprüche, **dadurch gekennzeichnet, dass** die Histamin-Provokationslösung Histamin in einem stabilisierenden Puffer in einer Konzentration zwischen 1 und 300 ng/ml oder zwischen 1 und 100 ng/ml, bevorzugt zwischen 3 und 60 ng/ml oder zwischen 6 und 30 ng/ml und besonders bevorzugt zwischen 17 und 23 ng/ml enthält.

5. Verfahren nach einem der vorangehenden Ansprüche, **dadurch gekennzeichnet, dass** der bestimmte Zeitraum der Inkubation der ersten und der zweiten Teilprobe 1 bis 50 Stunden oder 5 bis 50 Stunden, bevorzugt 12 bis 36 Stunden und besonders bevorzugt 20 bis 28 Stunden umfassen und die bestimmten ersten Inkubationsbedingungen eine Temperatur von 27°C bis 42°C, bevorzugt von 30°C bis 40°C und besonders bevorzugt von 34°C bis 38°C umfassen und die bestimmten zweiten Inkubationsbedingungen der zweite Teilprobe eine Temperatur von 2°C bis 27°C, bevorzugt von 3°C bis 10°C und besonders bevorzugt von 4°C bis 8°C umfassen.

6. Verfahren nach einem der vorangehenden Ansprüche, **dadurch gekennzeichnet, dass** der Verfahrensschritt der Modifikation des vorhandenen Histamins ein Acylieren umfasst.

7. Verfahren nach einem der vorangehenden Ansprüche, **dadurch gekennzeichnet, dass** das Verfahren einen zusätzlichen Schritt, nämlich die Zugabe von Peroxidase oder Katalase umfasst, insbesondere in einer Menge von 0,1 bis 10, vorzugsweise von 0,2 bis 5, noch mehr bevorzugt von 0,5 bis 2 Einheiten, insbesondere von 1 Einheit pro 100µl Probe.

8. Verfahren nach einem der vorangehenden Ansprüche, **dadurch gekennzeichnet, dass** die Konzentration an modifizierten Histamin mittels Immunoassay, insbesondere mittels Enzyme-linked Immunosorbent assay (ELISA) bestimmt wird.

9. Verfahren nach einem der vorangehenden Ansprüche, **dadurch gekennzeichnet, dass** die Konzentration an modifizierten Histamin mittels Hoch-Leistungs-Flüssig-Chromatographie (HPLC) und/oder Gaschromatographie (GS) bestimmt wird.

10. Verfahren nach einem der vorangehenden Ansprüche, **dadurch gekennzeichnet, dass** die Bestimmung der totalen Histamin-Abbaukapazität als Grundlage für die Diagnose und/oder als Überwachung einer therapeutischen Behandlung eines Krankheitsbildes eingesetzt wird, das auf einer defizitären totalen Histamin-Abbaukapazität basiert.

11. Verfahren nach einem der vorangehenden Ansprüche, **dadurch gekennzeichnet, dass** die Bestimmung der totalen Histamin-Abbaukapazität als Grundlage für die Risikoeinschätzung im Vorfeld einer Anästhesie bei einem Krankheitsbild eingesetzt wird, das auf einer defizitären totalen Histamin-Abbaukapazität basiert.

12. Verwendung eines Kit zur Bestimmung der totalen Histamin-Abbaukapazität in einer flüssigen biologischen Probe in einem Verfahren gemäß einem der Ansprüche 1 bis 11 umfassend zumindest:
- ein oder mehrere Probenaufnahmegefäße optional enthaltend eine Platte beschichtet mit modifiziertem Histamin, insbesondere acyliertem Histamin;
- eine Histamin-Provokationslösung enthaltend Histamin in einem stabilisierenden Puffer in einer Konzentration zwischen 1 bis 300 ng/ml oder zwischen 1 bis 100 ng/ml, bevorzugt zwischen 3 und 60 ng/ml oder zwischen 6 und 30 ng/ml und besonders bevorzugt zwischen 17 und 23 ng/ml,
- ein Histamin-Modifikationsreagens, insbesondere ein Histamin-Acylierungsreagens;
- Mittel zur Bestimmung von modifiziertem Histamin, insbesondere ELISA-basiert;
- optional Peroxidase oder Katalase, insbesondere in lyophilisierter Form,
- optional ein Histamin-Modifikationspuffer, insbesondere ein Histamin-Acylierungspuffer
- optional ein modifizierten Histamin-Standard, insbesondere acyliertes Histamin.

## Claims

1. A method for determining the total histamine degradation capacity in a liquid biological sample, the method **comprising the steps of:**
- equally dividing a provided amount of liquid biological sample into a first partial sample and a second partial sample;
- adding and mixing the first partial sample with a specific amount of histamine provocation solution containing histamine;
- incubating the first partial sample dosed with histamine provocation solution for a specific time period and in specific first incubation conditions, while at the same time incubating the second partial sample for the same specific time period as the first partial sample and in specific second incubation conditions;
- adding and mixing the second partial sample after its incubation with the same specific amount of histamine provocation solution containing histamine as previously employed for the first partial sample;
- extracting quantitatively equal fractions from the charges of the first and second partial samples containing provocation solution, and modifying the histamine still present in the charges;
- determining the absolute concentrations of modified histamine (histamine value) in the two partial samples;
- calculating the total histamine degradation capacity.

2. The method of claim 1, **characterized in that**
the total histamine degradation capacity is calculated based on the following formula: [(R - H) / (R/100)], where R = the concentration of the modified histamine (histamine value) of the second partial sample (reference value) and H = the concentration of the modified histamine (histamine value) of the first partial sample.

3. The method of claim 1 or 2, **characterized in that**
the liquid biological sample is whole blood, blood serum, blood plasma, cerebrospinal fluid, saliva, urine, and/or a homogenate of a biopsy tissue (tissue sample) or of a stool sample.

4. The method of any of the preceding claims, **characterized in that**
the histamine provocation solution contains histamine in a stabilizing buffer at a concentration of between 1 and 300 ng/ml or between 1 and 100 ng/ml, preferably between 3 and 60 ng/ml or between 6 and 30 ng/ml, and particularly preferably between 17 and 23 ng/ml.

5. The method of any of the preceding claims, **characterized in that**
the specific time period for incubation of the first and second partial samples comprises 1 to 50 hours or 5 to 50 hours, preferably 12 to 36 hours and particularly preferably 20 to 28 hours, and the specific first incubation conditions comprise a temperature of 27°C to 42°C, preferably of 30°C to 40°C and particularly preferably of 34°C to 38°C, and the specific second incubation conditions of the second partial sample comprise a temperature of 2°C to 27°C, preferably of 3°C to 10°C, and particularly preferably of 4°C to 8°C.

6. The method of any of the preceding claims, **characterized in that**
the step of modifying the histamine present comprises acylating.

7. The method of any of the preceding claims, **characterized in that**
the method comprises an additional step, i.e., adding peroxidase or catalase, particularly an amount of 0.1 to 10 units, preferably of 0.2 to 5 units, more preferably of 0.5 to 2 units, particularly of 1 unit, per 100µl of sample.

8. The method of any of the preceding claims, **characterized in that** the concentration of modified histamine is determined using an immunoassay, particularly using an enzyme-linked immunosorbent assay (ELISA).

9. The method of any of the preceding claims, **characterized in that**
the concentration of modified histamine is determined using high-performance liquid chromatography (HPLC) and/or gas chromatography (GS).

10. The method of any of the preceding claims, **characterized in that**
the determination of the total histamine degradation capacity is used as a basis for diagnosis and/or for monitoring a therapeutic treatment of a set of symptoms related to deficient total histamine degradation capacity.

11. The method of any of the preceding claims, **characterized in that**
the determination of the total histamine degradation capacity is used as a basis for risk assessment in preparation for anesthesia for a set of symptoms related to deficient total histamine degradation capacity.

12. Use of a kit for determining the total histamine degradation capacity in a liquid biological sample in a method according to any of claims 1 to 11, comprising at least:
- one or more sample receiving vessels optionally including a plate coated with modified histamine, particularly with acylated histamine;
- a histamine provocation solution containing histamine in a stabilizing buffer at a concentration of between 1 and 300 ng/ml or between 1 and 100 ng/ml, preferably between 3 and 60 ng/ml or between 6 and 30 ng/ml, and particularly preferably between 17 and 23 ng/ml,
- a histamine modification reagent, particularly a histamine acylation reagent;
- means for determining modified histamine, particularly based on ELISA;
- optionally peroxidase or catalase, particularly in lyophilized form,
- optionally a histamine modification buffer, particularly a histamine acylation buffer,
- optionally a modified histamine standard, particularly acylated histamine.

## Revendications

1. Procédé en vue de la détermination de la capacité totale de décomposition de l'histamine dans un échantillon biologique liquide **comprenant les étapes de** :
- répartition à parts égales d'une quantité fournie d'échantillon biologique liquide en un premier échantillon partiel et un second échantillon partiel ;
- addition et mélange du premier échantillon partiel avec une quantité déterminée de solution de provocation d'histamine contenant de l'histamine ;
- incubation du premier échantillon partiel additionné de solution de provocation d'histamine pendant une période déterminée et dans des premières conditions d'incubation déterminées, pendant que, simultanément, le deuxième échantillon partiel est incubé pendant la même période déterminée que le premier échantillon partiel et dans des secondes conditions d'incubation déterminées ;
- addition et mélange du second échantillon partiel après son incubation avec la même quantité déterminée de solution de provocation d'histamine contenant de l'histamine que celle utilisée auparavant pour le premier échantillon partiel ;
- prélèvement d'une fraction quantitativement égale des préparations des premier et second échantillons partiels contenant la solution de provocation et modification de l'histamine encore présente dans les préparations ;
- détermination de la concentration absolue de l'histamine modifiée (valeur histaminique) dans les deux échantillons partiels ;
- calcul de la capacité totale de décomposition de l'histamine.

2. Procédé selon la revendication 1, **caractérisé en ce que**
la capacité totale de décomposition de l'histamine est déterminée avec la formule suivante : [(R - H) / (R / 100)], sachant que
R = concentration de l'histamine modifiée (valeur histaminique) du second échantillon partiel (valeur de référence) et
H = concentration de l'histamine modifiée (valeur histaminique) du premier échantillon partiel.

3. Procédé selon la revendication 1 ou **2,caractérisé en ce que**
l'échantillon biologique liquide est du sang total, du sérum sanguin, du plasma sanguin, du liquide céphalo-rachidien, de la salive, de l'urine et/ou un homogénat d'un bioptat (échantillon de tissus) ou d'un échantillon de selles.

4. Procédé selon une quelconque des revendications précédentes, **caractérisé en ce que**
la solution de provocation d'histamine contient de l'histamine dans un tampon stabilisant à une concentration comprise entre 1 et 300 ng/ml ou entre 1 et 100 ng/ml, de préférence entre 3 et 60 ng/ml ou entre 6 et 30 ng/ml et de préférence particulière entre 17 et 23 ng/ml.

5. Procédé selon une quelconque des revendications précédentes, **caractérisé en ce que**
la période déterminée d'incubation des premier et second échantillons partiels comprend de 1 à 50 heures ou de 5 à 50 heures, de préférence de 12 à 36 heures et de préférence particulière de 20 à 28 heures et les premières conditions déterminées d'incubation comprennent une température de 27 °C à 42 °C, de préférence de 30 °C à 40 °C et de préférence particulière de 34 °C à 38 °C et les secondes conditions déterminées d'incubation du second échantillon partiel comprennent une température de 2 °C à 27 °C, de préférence de 3 °C à 10 °C et de préférence particulière de 4 °C à 8 °C.

6. Procédé selon une quelconque des revendications précédentes, **caractérisé en ce que**
l'étape de procédé de la modification de l'histamine présente comporte une acylation.

7. Procédé selon une quelconque des revendications précédentes, **caractérisé en ce que**
le procédé comporte une étape supplémentaire, à savoir l'addition de peroxydase ou de catalase, notamment en une quantité de 0,1 à 10, préférablement de 0,2 à 5, encore plus préférablement de 0,5 à 2 unités, notamment de 1 unité par 100 µl d'échantillon.

8. Procédé selon une quelconque des revendications précédentes, **caractérisé en ce que**
la concentration d'histamine modifiée est déterminée au moyen d'un immunoessai, notamment au moyen de l'essai ELISA (Enzyme-linked Immunosorbent assay).

9. Procédé selon une quelconque des revendications précédentes, **caractérisé en ce que**
la concentration d'histamine modifiée est déterminée au moyen d'une chromatographie liquide à haute performance (HPLC) et/ou d'une chromatographie gazeuse (GS).

10. Procédé selon une quelconque des revendications précédentes, **caractérisé en ce que**
la détermination de la capacité totale de décomposition de l'histamine est mise en œuvre comme base pour le diagnostic et/ou comme surveillance d'un traitement thérapeutique d'un tableau clinique qui se base sur une capacité totale déficitaire de décomposition de l'histamine.

11. Procédé selon une quelconque des revendications précédentes, **caractérisé en ce que**
la détermination de la capacité totale de décomposition de l'histamine est mise en œuvre comme base pour l'estimation des risques préalablement à une anesthésie dans le cas d'un tableau clinique qui se base sur une capacité totale de décomposition de l'histamine déficitaire.

12. Utilisation d'un kit en vue de la détermination de la capacité totale de décomposition de l'histamine dans un échantillon biologique liquide dans un procédé selon une quelconque des revendications 1 à 11 comportant au moins :
- un ou plusieurs récipients de collecte d'échantillons, contenant en option une plaque revêtue d'histamine modifiée, en particulier de l'histamine acylée ;
- une solution de provocation d'histamine contenant de l'histamine dans un tampon stabilisant dans une concentration comprise entre 1 à 300 ng/ml ou entre 1 à 100 ng/ml, de préférence entre 3 et 60 ng/ml ou entre 6 et 30 ng/ml et de préférence particulière entre 17 et 23 ng/ml,
- un réactif de modification de l'histamine, en particulier un réactif d'acylation de l'histamine ;
- des moyens en vue de la détermination de l'histamine modifiée, en particulier basés ELISA ;
- en option, de la peroxydase ou de la catalase, notamment sous forme lyophilisée,
- en option, un tampon de modification de l'histamine, notamment un tampon d'acylation de l'histamine,
- en option, un standard d'histamine modifié, en particulier de l'histamine acylée.
